# EUROPÄISCHE PATENTSCHRIFT

(11) **EP 0 805 803 B1**
(45) Veröffentlichungstag und Bekanntmachung des Hinweises auf die Patenterteilung: **30.10.2002**
(21) Anmeldenummer: 96901312.7
(22) Anmeldetag: 19.01.1996
(51) Int. Cl.: C07D 241/06

(54) **VERFAHREN ZUR HERSTELLUNG VON 1,4,5,6-TETRAHYDROPYRAZIN-2-CARBONSÄUREAMIDEN**
PROCESS FOR THE PRODUCTION OF 1,4,5,6-TETRAHYDROPYRAZINE-2-CARBOXYLIC ACID AMIDES
PROCEDE POUR LA PRODUCTION D'AMIDES D'ACIDE 1,4,5,6-TETRAHYDROPYRAZINE-2-CARBOXYLIQUE

(30) Priorität: 23.01.1995 CH 17895; 06.12.1995 CH 344395
(43) Veröffentlichungstag der Anmeldung: 12.11.1997
(73) Patentinhaber: Lonza AG, 3930 Visp (CH)
(72) Erfinder: BRIEDEN, Walter, CH-3902 Glis (CH); RODUIT, Jean-Paul, CH-3979 Grône (CH); FUCHS, Rudolf, CH-3950 Sion (CH)
(74) Vertreter: Weinhold, Peter, Dr.rer.nat.Dipl.Chem
(86) Internationale Anmeldenummer: EP9600223
(87) Internationale Veröffentlichungsnummer: WO96022981

(56) Entgegenhaltungen:
- TETRAHEDRON LETTERS, Bd. 36, Nr. 36, 1995 OXFORD GB, Seiten 6419-6422, K.ROSSEN ET AL. 'ASYMETRIC HYDROGENATION OF TETRAHYDROPYRAZINES:SYNTHESIS OF S-PIPERAZINE-2-TERT.BUTYLCARBOXAMIDE'

## Beschreibung

Die vorliegende Erfindung betrifft ein Verfahren zur Herstellung von 1,4,5,6-Tetrahydropyrazin-2-carbonsäureamiden der allgemeinen Formel 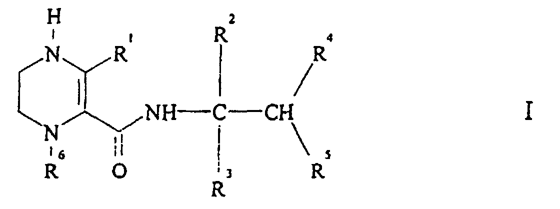 worin
R¹ Wasserstoff, ein C₁-C₃₀-Alkylrest, ein cycloaliphatischer Rest mit 3 bis 8 Ringgliedern, ein aromatischer Rest oder ein araliphatischer Rest mit 7 bis 12 Kohlenstoffatomen ist, sowie
(a) R² ein C₁-C₆-Alkylrest, ein Arylrest oder ein Arylalkylrest ist und R³, R⁴ und R⁵ unabhängig voneinander Wasserstoff, C₁-C₆-Alkylreste, Arylreste oder Arylalkylreste sind, oder
(b) R² zusammen mit R³ oder R⁴ und dem/den dazwischenliegenden Kohlenstoffatom(en) ein mono- oder polycyclisches alicyclisches System bildet, welches gegebenenfalls mit einer oder mehreren C₁-C₄-Alkylgruppen substituiert sein kann, und R⁵ und R⁴ oder R³ die vorstehend genannte Bedeutung haben,
und R⁶ Wasserstoff oder eine Gruppe der Formel R⁷-C(=O)- ist, wobei R⁷ Wasserstoff oder gegebenenfalls substituiertes C₁-C₆-Alkyl bedeutet.

Unter Alkylresten sind hier und im folgenden jeweils geradkettige oder verzweigte primäre, sekundäre oder tertiäre Alkylgruppen zu verstehen, also beispielsweise Methyl, Ethyl, Propyl, Isopropyl, Butyl, *sec*-Butyl, *tert*-Butyl, Isobutyl, Pentyl, Isopentyl, Neopentyl, Hexyl, Octyl usw.
Unter substituiertem Alkyl sind Alkoxyalkylgruppen oder Haloalkylgruppen zu verstehen, insbesondere Perfluoralkylgruppen wie beipielsweise Trifluormethyl.
Unter cycloaliphatischen Resten. sind Cycloalkylgruppen zu verstehen, also beispielsweise Cyclopropyl, Cyclobutyl, Cyclopentyl, Cyclohexyl usw.
Unter Arylresten beziehungsweise aromatischen Resten sind mono- oder bicyclische aromatische Gruppen zu verstehen, die gegebenenfalls mit C₁-C₄-Alkylgruppen oder Halogenen substituiert sein können, also beispielsweise Phenyl, 1-Naphthyl, 2-Naphthyl, o-Tolyl, *m*-Tolyl, *p*-Tolyl, Xylyle, Chlorphenyle usw.
Unter Arylalkylresten oder araliphatischen Resten sind entsprechend arylsubstituierte Alkylreste zu verstehen, insbesondere Benzyl, 1-Phenylethyl oder 2-Phenylethyl.

Es ist bekannt, dass Pyrazincarbonsäuren und deren Derivate wie Ester und Amide mit heterogenen Katalysatoren (z. B. Pd/C) hydriert werden können. Diese Hydrierung führt jedoch normalerweise zum entsprechenden Piperazinderivat, d. h. der heteroaromatische Ring wird vollständig hydriert. Eine teilweise Hydrierung zum Tetrahydropyrazinsystem wurde nur in Ausnahmefällen gefunden, wobei die Isolierung eines reinen Produktes im Fall der Tetrahydropyrazincarbonsäure*ester* Probleme bereitete und das entsprechende Amid auf diesem Weg nicht zugänglich war. (E. Felder et al., *Helv. Chim. Acta,* **1960,** *43*, 888-896).

Der vorliegenden Erfindung lag die Aufgabe zugrunde, einen technisch gangbaren Weg zu am Amidstickstoff substituierten 1,4,5,6-Tetrahydropyrazin-2-carbonsäureamiden aufzuzeigen. Diese bisher nicht bekannten Verbindungen sollten einen alternativen Zugang zu den entsprechenden Piperazincarbonsäureamiden ermöglichen, von denen beispielsweise das (*S*)-Piperazincarbonsäure-*tert*-butylamid Baustein eines Wirkstoffs zur Behandlung von AIDS ist (EP-A 541 168).

Erfindungsgemäss wird diese Aufgabe durch das Verfahren nach Patentanspruch I und die Verbindungen nach Patentanspruch 8 gelöst.

Es wurde gefunden, dass die 2-Cyan-1,4,5,6-tetrahydropyrazine der allgemeinen Formel 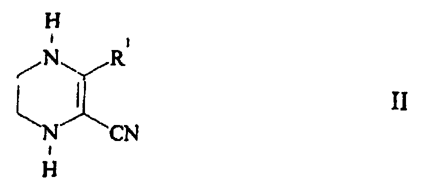 worin R¹ die oben genannte Bedeutung hat, in Gegenwart einer starken Säure mit Verbindungen der allgemeinen Formel 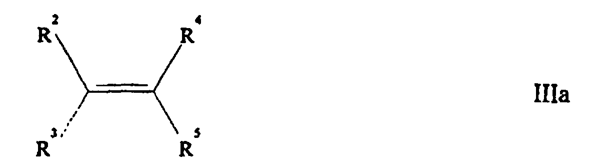 und/oder 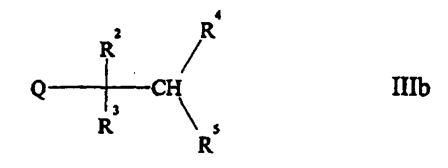 worin R², R³, R⁴ und R⁵ die oben genannten Bedeutungen haben und Q eine unter Bildung eines Carbeniumions abspaltbare Gruppe ist, eine Ritter-Reaktion eingehen. Hierbei bildet sich wahrscheinlich zunächst durch Addition des aus IIIa durch Protonierung und/oder aus IIIb durch Abspaltung der Gruppe Q gebildeten Carbeniumions an die Cyanogruppe ein Nitriliumsalz, das im weiteren Verlauf zum Amid hydrolysiert. Wird die Reaktion in Gegenwart einer Carbonsäure der Formel

R⁷―COOH IV

worin R⁷ die oben genannte Bedeutung hat, durchgeführt, dann wird ausserdem selektiv die Position 1 des Tetrahydropyrazinrings acyliert, so dass ein Produkt (I) erhalten wird, in welchem R⁶ = R⁷―C(=O)- ist. Ohne Zusatz einer Carbonsäure wird die entsprechende Verbindung mit R⁶ = H erhalten. Die Ausführungsform mit Carbonsäurezusatz ist bevorzugt, weil die Acylgruppe bei anschliessenden Synthesen als Schutzgruppe fungieren kann und gezielte Umsetzungen am Stickstoffatom in Position 4 erlaubt.
Besonders bevorzugt werden Carbonsäuren eingesetzt, in denen R⁷ Wasserstoff, eine C₁-C₃-Alkylgruppe oder eine C₁-C₃-Perfluoralkylgruppe ist, insbesondere Essigsäure (R⁷ = CH₃) oder Trifluoressigsäure (R⁷ = CF₃).

Als 2-Cyan-1,4,5,6-tetrahydropyrazine gemäss Formel II können sowohl solche mit Wasserstoff in Position 3 (R¹ = H) als auch solche mit C₁-C₃₀-Alkylresten, cycloaliphatischen Resten mit 3 bis 8 Ringgliedern, aromatischen Resten oder araliphatischen Resten mit 7 bis 12 Kohlenstoffatomen eingesetzt werden.
Diese Verbindungen sind nach dem in EP-A 0 175 364 beschriebenen Verfahren aus α-Dicarbonylverbindungen, Ethylendiamin und Cyanid leicht zugänglich.
Vorzugsweise wird die in Position 3 unsubstituierte Verbindung (R' = H) eingesetzt.

Es wurde ausserdem gefunden, dass entsprechende Verbindungen, die in Position I (am Stickstoffatom) bereits eine Acylgruppe tragen, überraschenderweise die erfindungsgemässe Reaktion gar nicht oder mindestens sehr schlecht eingehen, obwohl sie die gleichen Produkte liefern sollten. Andererseits wurde gefunden, dass auch unter drastischen Bedingungen (Überschuss an Carbonsäure, Gegenwart von Thionylchlorid) eine selektive N¹-Acylierung• stattfindet und praktisch keine N¹,N⁴-diacylierten Produkte erhalten werden.

Die 2-Cyan-1,4,5,6-tetrahydropyrazine (II) können sowohl als freie Basen als auch als Salze eingesetzt werden. Als Salze eignen sich insbesondere die Mono-Salze mit starken Säuren, beispielsweise die Hydrohalogenide, das Hydrogensulfat oder die Sulfonate. Diese Salze haben gegenüber den freien Basen auch den Vorteil einer besseren Lagerstabilität.
Besonders bevorzugt ist das Mono-Methansulfonat.

Als starke Säuren im Reaktionsmedium eignen sich entsprechend die sogenannten Mineralsäuren wie beispielsweise Schwefelsäure, Phosphorsäure und Perchlorsäure, Sulfonsäuren wie beispielsweise Methansulfonsäure, Benzolsulfonsäure oder polymere Sulfonsäuren (saure Ionenaustauscher), starke Carbonsäuren wie beispielsweise Ameisensäure oder Trifluoressigsäure oder auch Lewis-Säuren wie beispielsweise Bortrifluorid. Besonders bevorzugt ist Methansulfonsäure.

Als Verbindungen der Formeln IIIa und IIIb können sowohl Alkene (IIIa) als auch Verbindungen, welche durch Abspaltung einer Gruppe Q unter Einfluss einer starken Säure Carbeniumionen bilden können (IIIb) eingesetzt werden.

Als Alkene eignen sich sowohl offenkettige Alkene, in denen R² ein C₁-C₆-Alkylrest, ein Arylrest oder ein Arylalkylrest ist und R³, R⁴ und R⁵ unabhängig voneinander Wasserstoff, C₁-C₆-Alkyl, Aryl oder Arylalkyl sind, als auch cyclische Alkene. Bei den cyclischen Alkenen kann die Doppelbindung exocyclisch sein, so dass R² zusammen mit R³ und dem dazwischenliegenden Kohlenstoffatom ein mono- oder polycyclisches alicyclisches System bildet, oder endocyclisch, so dass R² zusammen mit R⁴ und den beiden dazwischenliegenden Kohlenstoffatomen ein mono- oder polycyclisches alicyclisches System bildet. Beispiele für offenkettige Alkene sind Propen, 1-Buten, 2-Buten, Isobuten, die verschiedenen isomeren Pentene und Hexene, Styrol, Alkylbenzol und Stilbene.

Alkene mit exocyclischer Doppelbindung sind beispielsweise Methylencyclohexan oder das bicyclische Camphen. Zu den Alkenen mit endocyclischer Doppelbindung zählen beispielsweise die Cycloalkene wie Cyclopenten, Cyclohexen und Cyclohepten.
Besonders bevorzugt als Alken (IIIa) ist das Isobuten (R² = R³ = CH³, R⁴ = R⁵ = H).

Von den Verbindungen (IIIb), die unter Einwirkung starker Säuren durch Abspaltung einer Gruppe Q Carbeniumionen liefern, sind insbesondere die Alkohole (Q = OH), sowie deren Derivate wie beispielsweise Ester (Q = Acyloxy) oder Ether (Q = Alkoxy) von Bedeutung. Beispielhaft sind hier Isopropyl- und *tert*-Butylalkohol, Methyl-*tert*-butylether, *tert*-Butylacetat oder Cyclohexanol zu erwähnen.

Es liegt selbstverständlich auch im Rahmen der Erfindung, Alkene (IIIa) zusammen mit entsprechenden Verbindungen der Formel IIIb einzusetzen, also beispielsweise Isobuten zusammen mit *tert*-Butylalkohol.

Die Reaktionsbedingungen sind abhängig von den eingesetzten Ausgangsmaterialien und ziemlich variabel. Als Lösungsmittel kann die Säure selbst dienen, also zum Beispiel Schwefelsäure oder die gegebenenfalls zugesetzte Carbonsäure (IV), es können aber auch noch weitere polare oder unpolare inerte Lösungsmittel zugesetzt werden.

Die Reaktionstemperatur liegt vorteilhaft im Bereich 0-50 °C, vorzugsweise bei 15-30 °C.

Es hat sich als vorteilhaft erwiesen, hohe Konzentrationen an Alken (IIIa) bzw. der Verbindung IIIb im Reaktionsgemisch zu vermeiden, um die Bildung von Polymeren und Oligomeren nicht zu begünstigen. Zu diesem Zweck wird das Alken bzw. die Verbindung IIIb nicht vorgelegt, sondern langsam entsprechend dem Reaktionsfortschritt zugegeben.

Die Aufarbeitung kann nach den üblichen Methoden erfolgen, beispielsweise durch Extraktion des gegebenenfalls neutralisierten Reaktionsgemisches. Häufig fällt das gebildete Produkt nach der basischen Aufarbeitung auch aus und kann durch Filtration oder Zentrifugation abgetrennt werden.

Die nachfolgenden Beispiele verdeutlichen die Durchführung des erfindungsgemässen Verfahrens.

### Beispiele:

### Beispiel 1

### 1-Acetyl-1,4,5,6-tetrahydropyrazin-2-carbonsäure-tert-butylamid

### (I; R¹ = R⁴ = R⁵ = H, R² = R³ = CH₃, R⁶ = Acetyl)

Zu 100 ml (1,75 mol) Essigsäure wurden bei Raumtemperatur langsam 70 g (0,77 mol) Methansulfonsäure und anschliessend 20,0 g (184 mmol) 2-Cyan-1,4,5,6-tetrahydropyrazin gegeben. In diese Mischung wurde bei 25 °C 23,0 g (410 mmol) Isobuten eingeleitet und das Gemisch 5 h gerührt.
Anschliessend wurde unter Kühlung mit 30%iger Natronlauge neutralisiert, wobei die Temperatur stets unter 30 °C gehalten wurde. Das auf pH 8-10 eingestellte Gemisch wurde dreimal mit je 200 ml Methylethylketon extrahiert. Die vereinigten organischen Phasen wurden über Magnesiumsulfat getrocknet und das Lösungsmittel abdestilliert. Der Rückstand (41,2 g) wurde in 80 ml Ethylacetat in der Hitze gelöst. Nach Abkühlung auf 20 °C wurden 500 ml Hexan zugegeben, weiter auf 0 °C abgekühlt, das ausgefallene Produkt nach 1 h abfiltriert und getrocknet.
- Ausbeute:: 32,6 g (79%) hellbeiges Pulver
- Schmp.:: 151,3-152,6°C
- ¹H NMR ([D₆]DMSO, 400 MHz): δ =: 1,27 (s, 9H);
1,89 (s, 3H);
3,06-3,12 (m, 2H);
3,31-3,40 (m, 2H);
6,46 (br. s, 1H);
6,59 (br. s, 1H);
6,80 (d, *J* = 7,2 Hz, 1H).

### Beispiel 2

### 1-Acetyl-1,4,5,6-tetrahydropyrazin-2-carbonsäure-tert-butylamid

Zu 30 ml Essigsäure und 20 ml Wasser wurden analog zu Beispiel 1 22 g (0,23 mol) Methansulfonsäure und 10 g (92 mmol) 2-Cyan-1,4,5,6-tetrahydropyrazin gegeben und 8,65 g (154 mmol) Isobuten eingeleitet.
Nach der Reaktion wurde das Gemisch bei 5 °C mit 119 g 30%iger Natronlauge neutralisiert und auf pH 11,6 eingestellt. Das ausgefallene Produkt wurde abfiltriert, mit 10 ml Eiswasser gewaschen und im Vakuum getrocknet.
Ausbeute: 28,9 g hellgelbes Pulver mit einem Gehalt von 58% (entspr. 80% d. Th.)

### Beispiel 3

### 1-Propionyl-1,4,5,6-tetrahydropyrazin-2-carbonsäure-tert-butylamid

### (I; R¹ = R⁴ = R⁵ = H, R² = R³ = CH₃, R⁶ = Propionyl)

Die Verbindung wurde analog zu Beispiel 1 unter Verwendung von Propionsäure anstelle von Essigsäure hergestellt.
- Ausbeute:: 75%
- Schmp.:: 172,4-172,6 °C
- ¹H NMR (CDCl₃, 400 MHz): δ =: 1,09 (t, *J* = 7,3 Hz, 3H);
1,37 (s, 9H);
2,39 (q, *J* = 7,3 Hz, 2H);
3,28 (m, 2H);
3,57 (m, 2H);
5,41 (br. s, 1H);
5,49 (br. s, 1H);
7,09 (d, *J* = 6,3 Hz, 1H).
- ¹³C NMR (CDCl₃, 100 MHz): δ =: 9,44 (CH₃);
27,55 (CH₂);
29,12 (CH₃);
38,29 (CH₂);
42,19 (CH₂);
50,92(C);
106,66(C);
132,20 (CH);
165,21 (C=O);
177,20 (C=O).

### Beispiel 4

### 1-Isobutyryl-1,4,5,6-tetrahydropyrazin-2-carbonsäure-tert-butylamid

### (I; R¹ = R⁴ = R⁵ = H, R² = R³ = CH₃, R⁶ = Isobutyryl)

Die Verbindung wurde analog zu Beispiel 1 unter Verwendung von Isobuttersäure anstelle von Essigsäure hergestellt.
- Ausbeute:: 48%
- Schmp.:: 180,0-184,4 °C
- ¹H NMR (CDCl₃, 400 MHz): δ =: 1,07 (d, *J* = 6,5 Hz, 6H);
1,37 (s, 9H);
2,82 (sept, *J* = 6,5 Hz, 1H);
3,27 (m, 2H);
3,6 (br. m, 2H);
4,94 (br. s, 1H);
5,48 (br. s, 1H);
7,12 (d, *J* = 6,3 Hz, 1H).
- ¹³C NMR (CDCl₃, 100 MHz): δ =: 19,36 (CH₃);
29,07 (CH₃);
32,50 (CH);
38,39 (CH₂);
42,42 (CH₂);
50,94 (C);
106,73 (C);
132,07 (CH);
165,21 (C=O);
180,85 (C=O).

### Beispiel 5

### 1-(Methoxyacetyl)-1,4,5,6-tetrahydropyrazin-2-carbonsäure-tert-butylamid

### (I; R¹ = R⁴ = R⁵ = H, R² = R³ = CH₃, R⁶ = Methoxyacetyl)

Die Verbindung wurde analog zu Beispiel 1 unter Verwendung von Methoxyessigsäure anstelle Essigsäure hergestellt.
- Ausbeute:: 52%
- Schmp.:: 185,5-189,9 °C
- ¹H NMR (CDCl₃, 400 MHz): δ =: 1,38 (s, 9H);
3,33 (m, 2H);
3,41 (s, 3H);
3,59 (m, 2H);
4,17 (s, 2H);
5,12 (br. s, 1H);
5,47 (br. s, 1H);
7,11 (d, *J* = 6,3 Hz, 1H).
- ¹³C NMR (CDCl₃, 100 MHz): δ =: 29,12 (CH₃);
38,27 (CH₂);
42,11 (CH₂);
51,09 (C);
59,31 (CH₃O);
70,86 (CH₃O);
105,48 (C);
132,72 (CH);
164,53 (C=O);
172,39 (C=O).

### Beispiel 6

### 1-Trifluoracetyl-1,4,5,6-tetrahydropyrazin-2-carbonsäure-tert-butylamid

### (I; R¹ = R⁴ = R⁵ = H, R² = R³ = CH₃, R⁶ = Trifluoracetyl ― aus 2-Cyan-1,4,5,6-tetrahydropyrazin-Methansulfonsäuresalz)

In einem 500 ml Kolben wurden 50 ml Trifluoressigsäure unter Argon vorgelegt. Bei 21 °C wurden 12,5 g Methansulfonsäure zugetropft und dann portionsweise 20 g 2-Cyan-1,4,5,6-tetrahydropyrazin-Methansulfonsäuresalz (97 mmol) zugegeben, wobei eine leichte Exothermie beobachtet wurde. Anschliessend wurden innerhalb 1 h bei 20 °C 10 g (178 mmol) Isobuten eingeleitet. Das Reaktionsgemisch wurde noch 2 h bei 20 °C gerührt, dann bei dieser Temperatur tropfenweise mit 13,3 g (111 mmol) Thionylchlorid versetzt und noch weitere 20 h gerührt. Dann wurden 250 ml Dichlormethan und portionsweise 25 g Natriumacetat zugegeben. Nach Filtration der rohen Lösung durch Celite® wurden 30 ml Wasser zugegeben und die Phasen getrennt. Die organische Phase wurde zur Trockne eingeengt. Das zurückbleibende Rohprodukt (27,88 g) wurde mit Ethylacetat/Methanol (4:1) an 300 g Kieselgel chromatographiert.
- Ausbeute (GC):: 29,2%.
- Schmp.:: 158-160 °C (aus n-Butylacetat).
- ¹H NMR (CDCl₃, 400 MHz): δ = 1,35 (s, 9H);
3,43 (br. s, 2H);
3,74 (br. s, 2H);
5,32 (br. s, 1H);
5,53 (br. s, 1H);
7,06 (d, *J* = 6 Hz, 1H).
- ¹³C NMR (CDCl₃, 100 MHz): δ = 28,9;
42,3;
42,8;
51,2;
105,9;
116,3 (*J*_{C-F} = 288 Hz);
133,2;
154,5 (*J*_{C-F} *=* 35 Hz);
163,7.
- MS: *m*/*z* =: 57 (100%), 279 (M⁺, 6,2%).

### Vergleichsbeispiel

### Versuch der Herstellung von 1-Acetyl-1,4,5,6-tetrahydropyrazin-2-carbonsäure-tertbutylamid aus 1-Acetyl-2-cyan-1,4,5,6-tetrahydropyrazin

### A) 1-Acetyl-2-cyan-1,4,5,6-tetrahydropyrazin

Zu 5,10 g (47 mmol) 2-Cyan-1,4,5,6-Tetrahydropyrazin wurden 10,0 ml (106 mmol) Essigsäureanhydrid und 8,0 ml (99 mmol) Pyridin gegeben und das Gemisch 18 h bei 20 °C gerührt. Das Reaktionsprodukt wurde wässrig aufgearbeitet und dreimal mit 100-200 ml Essigsäureethylester extrahiert. Die vereinigten organischen Extrakte wurden über Magnesiumsulfat getrocknet und eingeengt. Das Rohprodukt wurde dann mittels Flashchromatographie (Silicagel 40 × 3 cm, Essigester : Hexan = 4:1, R_{f} = 0,25) gereinigt. Die Fraktion mit R_{f} = 0,25 (5,82 g) wurde mit 40 ml Diethylether aufgenommen und der ausgefallene Feststoff abfiltriert und getrocknet.
- Schmp.:: 100,5-102,0 °C
- Ausbeute:: 5,62 g (80%) leicht gelblicher Feststoff

### NMR-Daten:

Die Rotation der Acetylgruppe um die Amidbindung ist stark behindert, so dass im NMR-Spektrum getrennte Signale für die beiden möglichen Konformeren (*E*- und *Z*-Form) im Intensitätsverhältnis 3:2 erhalten werden. Die beiden Konformeren sind hier mit A und B bezeichnet, wobei der Buchstabe A willkürlich dem in grösserer Menge vorliegenden Konformeren zugeordnet wurde.
¹H NMR ([D₆] DMSO, 400 MHz)
- Konformer A:δ =: 2,15 (s, 3H);
3,14-3,20 (m, 2H);
7,09 (d, *J* = 7,0 Hz, 1H);
7,46 (br. s, 1H).
- Konformer B: δ: 2,01 (s, 3H);
3,25-3,33 (m, 2H);
6,90 (d, *J* = 7,0 Hz, 1H);
7,01 (br. s, 1H).
- Konformer A + B: δ =: 3,44-3,56 (m, 2 + 2 H)
¹³C NMR ([D₆] DMSO, 100 MHz)
- Konformer A: δ =: 22,38 (CH₃);
80,67 (C);
119,48 (C≡N);
138,59 (CH);
168,50 (C=O).
- Konformer B: δ =: 20,97 (CH₃);
81,94 (C);
118,35 (C≡N);
137,16 (CH);
166,65 (C=O).
- Zuordnung unklar: δ =: 36,17 (CH₂);
41,45 (CH₂);
41,69 (CH₂);
41,74 (CH₂).

### B) Versuchte Umsetzung mit Isobuten

Zu einer Lösung von 12,73 g 98%iger Schwefelsäure (130 mmol) in 50 ml Eisessig wurde bei 20 °C 4,91 g 1-Acetyl-2-cyan-1,4,5,6-tetrahydropyrazin (32,5 mmol) zugegeben. Innerhalb von 3 h wurden bei konstanter Temperatur 3,62 g Isobuten (64,5 mmol) eingeleitet, anschliessend wurde das Reaktionsgemisch noch 3 h gerührt.

Die dunkelbraune Suspension wurde auf 106 g Eis gegossen und bei T < 20 °C mit 30%iger Natronlauge auf pH 8 gestellt. Ein feiner Niederschlag (Na₂SO₄) wurde abfiltriert, das Filtrat wurde über Nacht mit Dichlormethan kontinuierlich extrahiert. Durch Eindampfen der organischen Phase wurde nur wenig schwarzer Feststoff isoliert. Die wässrige Phase wurde eingeengt. Der feste Rückstand wurde mit 80 ml siedendem Ethanol aufgeschlämmt, wobei ca. 2/3 der Menge in Lösung ging. Nach dem Eindampfen wurde 6,8 g oranger Feststoff erhalten. Dieser wurde mit heissem Isopropanol aufgenommen, wobei der unlösliche Teil wieder abfiltriert wurde. Aus der Isopropanol-Lösung kristallisierte 3,52 g 1-Acetyl-1,4,5,6-tetrahydropyrazin-2-carboxamid als beiger Feststoff (64% Ausbeute).
- ¹H NMR ([D₆]DMSO, 400 MHz): δ =: 1,92 (s, 3H);
3,10 (m, 2H);
3,35 (m, 2H);
6,60 (br. s, 2H);
6,72 (br. s, 1H);
7,00 (d, *J* = 6,3 Hz, 1H).
- ¹³C NMR ([D₆]DMSO, 100 MHz): δ =: 25,39 (CH₃);
36,89 (CH₂);
41,51 (CH₂);
105,37 (C);
131,96 (CH);
166,66 (C=O);
170,33 (C=O).

## Patentansprüche

1. Verfahren zur Herstellung von 1,4,5,6-Tetrahydropyrazin-2-carbonsäureamiden der allgemeinen Formel 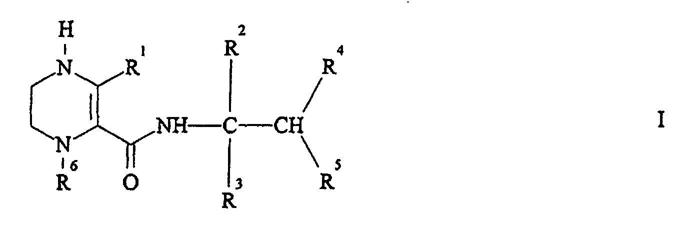 worin R¹ Wasserstoff, ein C₁-C₃₀-Alkylrest, eine Cycloalkylgruppe mit 3 bis 8 Ringgliedern, eine mono- oder bicyclische aromatische Gruppe, welche gegebenenfalls mit C₁-C₄-Alkylgruppen oder Halogenen substituiert ist, oder ein mit einer mono- oder bicyclischen aromatischen Gruppe substituierter Alkylrest mit 7 bis 12 Kohlenstoffatomen ist,
(a) R² ein C₁-C₆-Alkylrest, eine mono- oder bicyclische aromatische Gruppe, welche gegebenenfalls mit C₁-C₄-Alkylgruppen oder Halogenen substituiert ist, Benzyl, 1-Phenylethyl oder 2-Phenylethyl ist und R³, R⁴ und R⁵ unabhängig voneinander Wasserstoff, C₁-C₆-Alkylreste, mono- oder bicyclische aromatische Gruppen, welche gegebenenfalls mit C₁-C₄-Alkylgruppen oder Halogenen substituiert sind, Benzyl, 1-Phenylethyl oder 2-Phenylethyl sind, oder
(b) R² zusammen mit R³ oder R⁴ und dem / den dazwischenliegenden Kohlenstoffatom(en) ein mono- oder polycyclisches alicyclisches System bildet, welches gegebenenfalls mit einer oder mehreren C₁-C₄-Alkylgruppen substituiert sein kann, und R⁵ und R⁴ oder R³ die vorstehend genannte Bedeutung haben, und R⁶ Wasserstoff oder eine Gruppe der Formel R⁷―C(=O)― ist, wobei R⁷ Wasserstoff, C₁-C₆-Alkyl, Alkoxy-C₁-C₆-alkyl oder Halo-C₁-C₆-alkyl bedeutet,
**dadurch gekennzeichnet, dass** ein 2-Cyan-1,4,5,6-tetrahydropyrazin der allgemeinen Formel 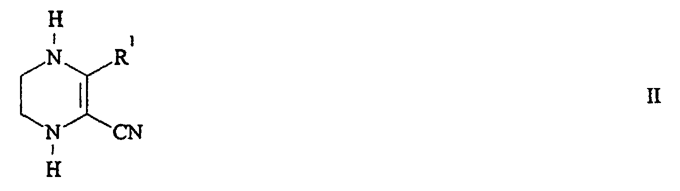 worin R¹ die oben genannte Bedeutung hat, oder ein Salz davon in Gegenwart einer starken Säure mit einer Verbindung der allgemeinen Formel 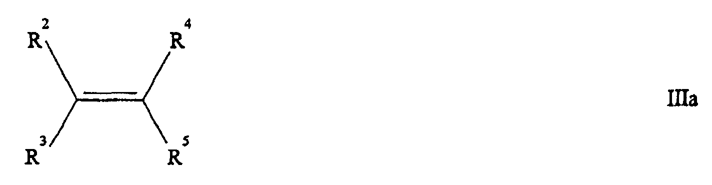 und / oder 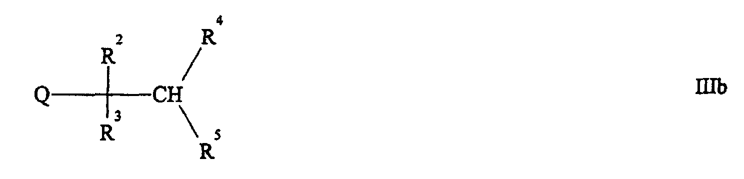 worin R², R³, R⁴ und R⁵ die oben genannte Bedeutung haben und Q eine unter Bildung eines Carbeniumions abspaltbare Gruppe ist, und gegebenenfalls einer Carbonsäure der Formel
R⁷―COOH IV
worin R⁷ die oben genannte Bedeutung hat, umgesetzt und die gebildete Nitriliumverbindung zum Amid (I) hydrolysiert wird.

2. Verfahren nach Anspruch 1, **dadurch gekennzeichnet, dass** die Umsetzung des 2-Cyan-1,4,5,6-tetrahydropyrazins (II) mit der Verbindung IIIa und/oder IIIb in Gegenwart einer Carbonsäure (IV) durchgeführt und ein 1-Acyl-1,4,5,6-tetrahydropyrazin-2-carbonsäurearnid (I), worin R⁶ eine Gruppe der Formel R⁷―C(=O)― ist, erhalten wird.

3. Verfahren nach Anspruch 2, **dadurch gekennzeichnet, dass** als Carbonsäure Essigsäure oder Trifluoressigsäure eingesetzt wird.

4. Verfahren nach einem der Ansprüche 1 bis 3, **dadurch gekennzeichnet, dass** als 2-Cyan-1,4,5,6-tetrahydropyrazin das in Position 3 unsubstituierte (R¹ = H) 2-Cyan-1,4,5,6-tetrahydropyrazin eingesetzt wird.

5. Verfahren nach Anspruch 4, **dadurch gekennzeichnet, dass** das 2-Cyan-1,4,5,6-tetrahydropyrazin in Form des Mono-methansulfonats eingesetzt wird.

6. Verfahren nach einem oder mehreren der Ansprüche 1 bis 5, **dadurch gekennzeichnet, dass** als starke Säure Methansulfonsäure eingesetzt wird.

7. Verfahren nach einem oder mehreren der Ansprüche 1 bis 6, **dadurch gekennzeichnet, dass** als Verbindung der Formel IIIa Isobuten und/oder als Verbindung der Formel IIIb *tert*-Butylalkohol eingesetzt wird.

8. Verbindungen der Formel 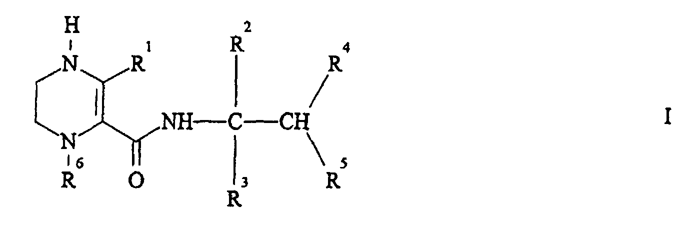 worin R¹ Wasserstoff, ein C₁-C₃₀-Alkylrest, eine Cycloalkylgruppe mit 3 bis 8 Ringgliedern, eine mono- oder bicyclische aromatische Gruppe, welche gegebenenfalls mit C₁-C₄-Alkylgruppen oder Halogenen substituiert ist, oder ein mit einer mono- oder bicyclischen aromatischen Gruppe substituierter Alkylrest mit 7 bis 12 Kohlenstoffatomen ist,
(a) R² ein C₁-C₆-Alkylrest, eine mono- oder bicyclische aromatische Gruppe, welche gegebenenfalls mit C₁-C₄-Alkylgruppen oder Halogenen substituiert ist, Benzyl, 1-Phenylethyl oder 2-Phenylethyl ist und R³, R⁴ und R⁵ unabhängig voneinander Wasserstoff, C₁-C₆-Alkylreste, mono- oder bicyclische aromatische Gruppen, welche gegebenenfalls mit C₁-C₄-Alkylgruppen oder Halogenen substituiert sind, Benzyl, 1-Phenylethyl oder 2-Phenylethyl sind, oder
(b) R² zusammen mit R³ oder R⁴ und dem / den dazwischenliegenden Kohlenstoffatom(en) ein mono- oder polycyclisches alicyclisches System bildet, welches gegebenenfalls mit einer oder mehreren C₁-C₄-Alkylgruppen substituiert sein kann, und R⁵ und R⁴ oder R³ die vorstehend genannte Bedeutung haben,
und R⁶ Wasserstoff oder eine Gruppe der Formel R⁷―C(=O)― ist, wobei R⁷ Wasserstoff, C₁-C₆-Alkyl, Alkoxy-C₁-C₆-alkyl oder Halo-C₁-C₆-alkyl bedeutet.

9. Verbindungen nach Anspruch 8, **dadurch gekennzeichnet, dass** R¹ Wasserstoff ist.

10. Verbindungen nach Anspruch 8 oder 9, **dadurch gekennzeichnet, dass** R⁶ eine Gruppe der Formel R⁷―C(=O)― ist, wobei R⁷ die in Anspruch 8 genannte Bedeutung hat.

11. Verbindungen nach einem der Ansprüche 8 bis 10, **dadurch gekennzeichnet, dass** R² und R³ Methyl und R⁴ und R⁵ Wasserstoff sind.

12. Verbindungen nach einem der Ansprüche 10 bis 11, **dadurch gekennzeichnet, dass** R⁷ Wasserstoff, C₁-C₃-Alkyl oder C₁-C₃-Perfluoralkyl ist.

13. Verbindungen nach Anspruch 12, **dadurch gekennzeichnet, dass** R⁷ Methyl oder Trifluormethyl ist.

## Claims

1. Process for the preparation of 1,4,5,6-tetrahydropyrazine-2-carboxylic acid amides of the general formula 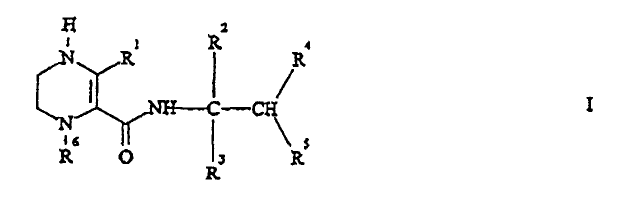 wherein R¹ is hydrogen, a C₁-C₃₀-alkyl radical, a cycloalkyl group having 3 to 8 ring members, a mono- or bicyclic aromatic group, which is optionally substituted by C₁-C₄-alkyl groups or halogens, or an alkyl radical having 7 to 12 carbon atoms which is substituted by a mono- or bicyclic aromatic group,
(a) R² is a C₁-C₆-alkyl radical, a mono- or bicyclic aromatic group, which is optionally substituted by C₁-C₄-alkyl groups or halogens, benzyl, 1-phenylethyl or 2-phenylethyl and R³, R⁴ and R⁵ independently of one another are hydrogen, C₁-C₆-alkyl radicals, mono- or bicyclic aromatic groups, which are optionally substituted by C₁-C₄-alkyl groups or halogens, benzyl, 1-phenylethyl or 2-phenylethyl, or
(b) R², together with R³ or R⁴ and the carbon atom(s) in between, forms a mono- or polycyclic alicyclic system, which can optionally be substituted by one or more C₁-C₄-alkyl groups, and R⁵ and R⁴ or R³ have the abovementioned meaning,
and R⁶ is hydrogen or a group of the formula R⁷-C(=O)-, wherein R⁷ denotes hydrogen, C₁-C₆-alkyl, alkoxy-C₁-C₆-alkyl or halo-C₁-C₆-alkyl,
**characterized in that** a 2-cyano-1,4,5,6-tetrahydropyrazine of the general formula 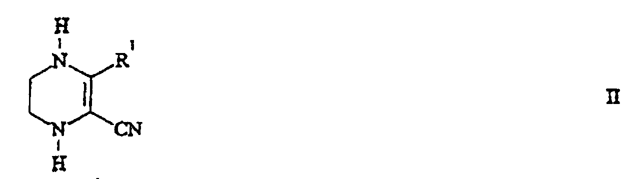 wherein R¹ has the abovementioned meaning, or a salt thereof, is reacted in the presence of a strong acid with a compound of the general formula 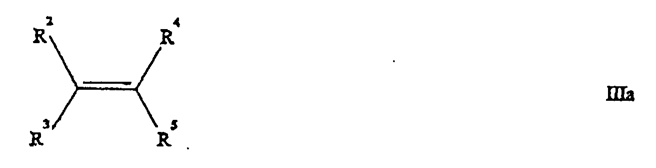 and/or 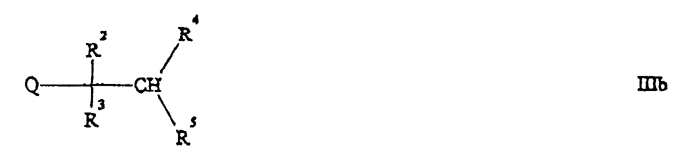 wherein R², R³, R⁴ and R⁵ have the abovementioned meaning and Q is a group which can be split off to form a carbenium ion,
and optionally a carboxylic acid of the formula
**R**^{**7**}**―COOH IV**
wherein R⁷ has the abovementioned meaning, and the nitrilium compound formed is hydrolysed to the amide (I).

2. Process according to claim 1, **characterized in that** the reaction of the 2-cyano-1,4,5,6-tetrahydropyrazine (II) with the compound IIIa and/or IIIb is carried out in the presence of a carboxylic acid (IV) and a 1-acyl-1,4,5,6-tetrahydropyrazine-2-carboxylic acid amide (I) wherein R⁶ is a group of the formula R⁷-C(=O)- is obtained.

3. Process according to claim 2, **characterized in that** acetic acid or trifluoroacetic acid is employed as the carboxylic acid.

4. Process according to one of claims 1 to 3,
**characterized in that** 2-cyano-1,4,5,6-tetrahydropyrazine, which is unsubstituted in position 3 (R¹ = H), is employed as the 2-cyano-1,4,5,6-tetrahydropyrazine.

5. Process according to claim 4, **characterized in that** the 2-cyano-1,4,5,6-tetrahydropyrazine is employed in the form of the mono-methanesulfonate.

6. Process according to one or more of claims 1 to 5,
**characterized in that** methanesulfonic acid is employed as the strong acid.

7. Process according to one or more of claims 1 to 6,
**characterized in that** isobutene is employed as the compound of the formula IIIa and/or *tert*-butyl alcohol is employed as the compound of the formula IIIb.

8. Compounds of the formula 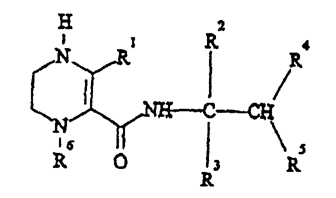 wherein R¹ is hydrogen, a C₁-C₃₀-alkyl radical, a cycloalkyl group having 3 to 8 ring members, a mono-or bicyclic aromatic group, which is optionally substituted by C₁-C₄-alkyl groups or halogens, or an alkyl radical having 7 to 12 carbon atoms which is substituted by a mono- or bicyclic aromatic group,
(a) R² is a C₁-C₆-alkyl radical, a mono- or bicyclic aromatic group, which is optionally substituted by C₁-C₄-alkyl groups or halogens, benzyl, 1-phenylethyl or 2-phenylethyl and R³, R⁴ and R⁵ independently of one another are hydrogen, C₁-C₆-alkyl radicals, mono- or bicyclic aromatic groups, which are optionally substituted by C₁-C₄-alkyl groups or halogens, benzyl, 1-phenylethyl or 2-phenylethyl, or
(b) R², together with R³ or R⁴ and the carbon atom(s) in between, forms a mono- or polycyclic alicyclic system, which can optionally be substituted by one or more C₁-C₄-alkyl groups, and R⁵ and R⁴ or R³ have the abovementioned meaning,
and R⁶ is hydrogen or a group of the formula R⁷-C(=O)-, wherein R⁷ denotes hydrogen, C₁-C₆-alkyl, alkoxy-C₁-C₆-alkyl or halo-C₁-C₆-alkyl.

9. Compounds according to claim 8, **characterized in that** R¹ is hydrogen.

10. Compounds according to claim 8 or 9, **characterized in that** R⁶ is a group of the formula R⁷-C(=O)-, wherein R⁷ has the meaning given in claim 8.

11. Compounds according to one of claims 8 to 10, **characterized in that** R² and R³ are methyl and R⁴ and R⁵ are hydrogen.

12. Compounds according to one of claims 10 to 11,
**characterized in that** R⁷ is hydrogen, C₁-C₃-alkyl or C₁-C₃-perfluoroalkyl.

13. Compounds according to claim 12, **characterized in that** R⁷ is methyl or trifluoromethyl.

## Revendications

1. Procédé de préparation d'amides d'acide 1,4,5,6-tétrahydropyrazine-2-carboxylique de formule générale 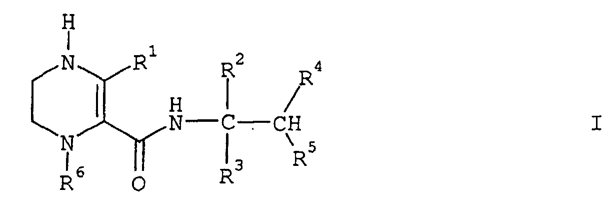 où R¹ est l'hydrogène, un reste alkyle en C₁-C₃₀, un groupe cycloalkyle à 3 à 8 chaînons, un groupe aromatique mono- ou bicyclique, qui est éventuellement substitué par des groupes alkyle en C₁-C₄ ou des halogènes, ou un reste alkyle à 7 à 12 atomes de carbone substitué par un groupe aromatique mono- ou bicyclique,
(a) R² est un reste alkyle en C₁-C₆, un groupe aromatique mono- ou bicyclique, qui est éventuellement substitué par des groupes alkyle en C₁-C₄ ou des halogènes, benzyle, 1-phényléthyle ou 2-phényléthyle et R³, R⁴ et R⁵ sont indépendamment les uns des autres l'hydrogène, des restes alkyle en C₁-C₆, des groupes aromatiques mono- ou bicycliques qui sont éventuellement substitués par des groupes alkyle en C₁-C₄ ou des halogènes, benzyle, 1-phényléthyle ou 2-phényléthyle, ou
(b) R² forme avec R³ ou R⁴ et le ou les atomes de carbone situés entre eux un système alicyclique mono- ou polycyclique qui peut éventuellement être substitué par un ou plusieurs groupes alkyle en C₁-C₄, et R⁵ et R⁴ ou R³ ont la signification citée précédemment, et R⁶ est l'hydrogène ou un groupe de formule R⁷-C(=O)- où R⁷ représente l'hydrogène, alkyle en C₁-C₆, alcoxy-alkyle en C₁-C₆ ou halogéno-alkyle en C₁-C₆,
**caractérisé en ce qu'**une 2-cyano-1,4,5,6-tétrahydropyrazine de formule générale 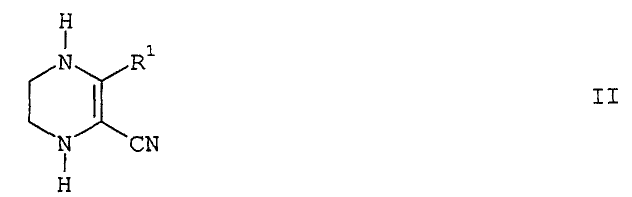 où R¹ a la signification citée ci-dessus, ou un sel de celle-ci, est mise à réagir en présence d'un acide fort avec un composé de formule générale 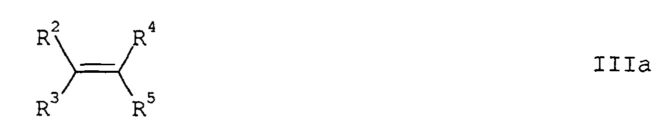 et/ou 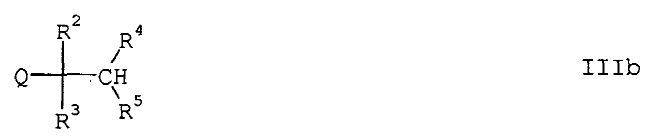 où R², R³, R⁴ et R⁵ ont la signification citée ci-dessus et Q est un groupe clivable avec formation d'un ion carbénium,
et éventuellement avec un acide carboxylique de formule
R⁷-COOH IV
où R⁷ a la signification citée ci-dessus, et le composé nitrilium formé est hydrolysé en l'amide (I).

2. Procédé selon la revendication 1 **caractérisé en ce que** la réaction de la 2-cyano-1,4,5,6-tétrahydropyrazine (II) avec le composé IIIa et/ou IIIb est conduite en présence d'un acide carboxylique (IV) et un amide d'acide 1-acyl-1,4,5,6-tétrahydropyrazine-2-carboxylique (I), où R⁶ est un groupe de formule R⁷-C(=O)-, est obtenu.

3. Procédé selon la revendication 2 **caractérisé en ce que** l'on utilise l'acide acétique ou l'acide trifluoroacétique comme acide carboxylique.

4. Procédé selon l'une des revendications 1 à 3 **caractérisé en ce que** l'on utilise comme 2-cyano-1,4,5,6-tétrahydropyrazine la 2-cyano-1,4,5,6-tétrahydropyrazine non substituée en position 3 (R¹ = H).

5. Procédé selon la revendication 4 **caractérisé en ce que** l'on utilise la 2-cyano-1,4,5,6-tétrahydropyrazine sous forme du monométhanesulfonate.

6. Procédé selon une ou plusieurs des revendications 1 à 5 **caractérisé en ce que** l'on utilise l'acide méthanesulfonique comme acide fort.

7. Procédé selon une ou plusieurs des revendications 1 à 6 **caractérisé en ce que** l'on utilise l'isobutène comme composé de formule IIIa et/ou l'alcool *tert*-butylique comme composé de formule IIIb.

8. Composés de formule 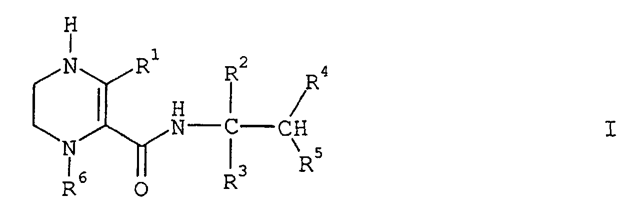 où R¹ est l'hydrogène, un reste alkyle en C₁-C₃₀, un groupe cycloalkyle à 3 à 8 chaînons, un groupe aromatique mono- ou bicyclique, qui est éventuellement substitué par des groupes alkyle en C₁-C₄ ou des halogènes, ou un reste alkyle à 7 à 12 atomes de carbone substitué par un groupe aromatique mono- ou bicyclique,
(a) R² est un reste alkyle en C₁-C₆, un groupe aromatique mono- ou bicyclique, qui est éventuellement substitué par des groupes alkyle en C₁-C₄ ou des halogènes, benzyle, 1-phényléthyle ou 2-phényléthyle et R³, R⁴ et R⁵ sont indépendamment les uns des autres l'hydrogène, des restes alkyle en C₁-C₆, des groupes aromatiques mono- ou bicycliques qui sont éventuellement substitués par des groupes alkyle en C₁-C₄ ou des halogènes, benzyle, 1-phényléthyle ou 2-phényléthyle, ou
(b) R² forme avec R³ ou R⁴ et le ou les atomes de carbone situés entre eux un système alicyclique mono- ou polycyclique qui peut éventuellement être substitué par un ou plusieurs groupes alkyle en C₁-C₄, et R⁵ et R⁴ ou R³ ont la signification citée précédemment, et R⁶ est l'hydrogène ou un groupe de formule R⁷-C(=O)- où R⁷ représente l'hydrogène, alkyle en C₁-C₆, alcoxy-alkyle en C₁-C₆ ou halogéno-alkyle en C₁-C₆.

9. Composés selon la revendication 8 **caractérisés en ce que** R¹ est l'hydrogène.

10. Composés selon la revendication 8 ou 9 **caractérisés en ce que** R⁶ est un groupe de formule R⁷-C(=O)- où R⁷ a la signification citée dans la revendication 8.

11. Composés selon l'une des revendications 8 à 10 **caractérisés en ce que** R² et R³ sont méthyle et R⁴ et R⁵ sont l'hydrogène.

12. Composés selon l'une des revendications 10 à 11 **caractérisés en ce que** R⁷ est l'hydrogène, alkyle en C₁-C₃ ou perfluoroalkyle en C₁-C₃.

13. Composés selon la revendication 12 **caractérisés en ce que** R⁷ est méthyle ou trifluorométhyle.
